# EUROPEAN PATENT APPLICATION

(11) **EP 4 095 237 A1**
(43) Date of publication of application: **30.11.2022**
(21) Application number: 21744617.8
(22) Date of filing: 19.01.2021
(51) Int. Cl.: C12N 5/077

(54) **METHOD FOR PRODUCING THREE-DIMENSIONAL CELL STRUCTURE**

(30) Priority: 20.01.2020 JP 2020006587
(71) Applicant: TOPPAN INC., Tokyo 110-0016 (JP)
(72) Inventor: HIRAOKA, Yasuyuki, Tokyo 110-0016 (JP)
(74) Representative: Plasseraud IP
(86) International application number: PCT/JP2021/001593
(87) International publication number: WO 2021/149661

(57) **Abstract**

This method of producing a three-dimensional cell structure, includes: mixing a cell cluster including at least an endothelial cell, an extracellular matrix component, and a polymer electrolyte to obtain a mixture; removing a liquid part from the mixture to obtain a cell aggregate; and culturing the cell aggregate in a medium to obtain a three-dimensional cell structure with a thickness greater than 150 µm and having a vascular network, characterized in that the extracellular matrix component is collagen or a collagen analog, and the polymer electrolyte is heparin or a heparin analog having a final concentration of 0.001 mg/mL or higher in the mixture.

## Description

### [Technical Field]

The present invention relates to a method of producing a three-dimensional cell structure.

The present application claims the benefit of priority from Japanese Patent Application No. 2020-006587 filed on January 20, 2020, the contents of which are incorporated herein by reference.

### [Background Art]

In recent years, the superiority of using three-dimensional cell structures organized threedimensionally over cells grown on a flat plate has been shown not only in regenerative medicine but also in assay systems for drugs that require an environment close to a living body. Various techniques for constructing a three-dimensional cell structure in vitro have been developed. For example, a method of forming a cell mass on a surface substrate to which cells cannot adhere, a method of forming a cell mass in a droplet, a method of accumulating cells on a permeable membrane, and the like have been developed.

In order to maintain such cell organization, an extracellular matrix (may also be referred to as ECM hereinafter) such as collagen produced by the living body itself is required for cell-cell binding and scaffold formation. Therefore, adding an ECM from the outside when constructing a cell tissue artificially has been considered.

For example, PTL 1 discloses a method in which cells isolated by enzyme treatment or the like are brought into contact with collagen, which is a typical ECM, and a water-soluble polymer having a cytoprotective effect, or the like, and then cultured as a three-dimensional aggregate. According to this method, a water-soluble polymer gel is formed around the cells while they are being cultured.

PTL 2 discloses a method in which a three-dimensional cell structure is formed by preparing cell sheets using culture dishes having poly(N-isopropylacrylamide) (also referred to as PIPAAm), which is a temperature-responsive resin, immobilized on the surface, and laminating the prepared cell sheets.

PTL 3 discloses a method in which a three-dimensional cell structure is constructed by repeating a step of forming a cell layer and a step of bringing the formed cell layer into contact alternately with a solution containing a first substance and a solution containing a second substance, and continuously stacking cell layers via an ECM having a nanometer-sized thickness. It is stated that, since there is no need to peel off a single-layer cell sheet or laminate the peeled cell sheets, a three-dimensional cell structure can be produced with great reproducibility and efficiency.

PTL 4 discloses a method in which a three-dimensional cell structure is constructed by preparing coated cells whose entire surfaces are coated with adhesive membranes and binding the cells together via the adhesive membranes.

As described above, a number of methods have been proposed as techniques for producing three-dimensional cell structures. However, even if a three-dimensional cell structure could be temporarily formed by any of the techniques, it cannot be said that it successfully imitates a structure that is truly close to a living body unless a vascular network can be reproduced inside the three-dimensional cell structure. In particular, in the case of a three-dimensional cell structure, it is required that the vascular network is well formed in order to supply necessary nutrients to the cells inside the three-dimensional cell structure.

In general, to form a vascular network, vascular endothelial cells are added to the cell cluster forming the three-dimensional cell structure. However, just including vascular endothelial cells in the three-dimensional cell structure does not guarantee the formation of a vascular network (blood vessel network), and the environment around the vascular endothelial cells is important for the formation of a blood vessel network.

Various factors such as various growth factors including vascular endothelial growth factor (also referred to as VEGF) are known as factors that affect the formation of a blood vessel network inside a three-dimensional cell structure, and, as one of those factors, it has been reported that the mechanical properties of the ECM affect the formation of a blood vessel network (see NPL 1).

The inventors of the present invention have previously developed a technique of producing a three-dimensional cell structure, including: a step A of acquiring a mixture including cells suspended in a solution containing at least a cationic buffer solution, an ECM, and a polymer electrolyte; a step B of collecting the cells from the obtained mixture to form a cell aggregate on a substrate; and a step C of culturing the cells to obtain a three-dimensional cell structure (see PTL 5).

### [Citation List]

### [Patent Literature]

[PTL 1] JP 2824081 B
[PTL 2] WO 2002/008387
[PTL 3] JP 4919464 B
[PTL 4] JP 5850419 B
[PTL 5] JP 6427836 B

### [Non-Patent Literature]

[NPL 1] Rouwkema J. and Khademhosseini A., Vascularization and Angiogenesis in Tissue Engineering: Beyond Creating Static Networks, Trends Biotechnol, 34 (9), 733-745, 2016.

### [Summary of the Invention]

### [Technical Problem]

The method described in PTL 5 is a technique that is applicable without being significantly limited by the cell type, shows good performance in forming a vascular network when the cells include endothelial cells, and is also effective in forming a layered configuration.

However, for easier acquisition of a thick three-dimensional cell structure when the ECM includes collagen, there is room for further improvement.

Therefore, an object of the present invention is to provide a technique for producing a three-dimensional cell structure having a sufficient thickness and a vascular network.

### [Solution to Problem]

The present invention has the following modes.
[1] A method of producing a three-dimensional cell structure, including: mixing a cell cluster including at least an endothelial cell, an extracellular matrix component, and a polymer electrolyte to obtain a mixture; removing a liquid part from the mixture to obtain a cell aggregate; and culturing the cell aggregate in a medium to obtain a three-dimensional cell structure with a thickness of greater than 150 µm and having a vascular network, characterized in that the extracellular matrix component is collagen or a collagen analog, and the polymer electrolyte is heparin or a heparin analog having a final concentration of 0.001 mg/mL or higher in the mixture.
[2] The production method according to [1], characterized in that, in the culturing of the cell aggregate in a medium, the cell aggregate is seeded at a cell density of 1,000 cells/mm² to 1,000,000 cells/mm² with respect to the area of a culture vessel.
[3] The production method according to [1] or [2], characterized in that a total length of vessels constituting the vascular network is 5,000 µm/mm² or more.
[4] The production method according to any one of [1] to [3], characterized in that the endothelial cell is a vascular endothelial cell.
[5] The production method according to any one of [1] to [4], characterized in that the cell aggregate further includes a fibroblast.
[6] The production method according to any one of [1] to [5], wherein a pH of the mixture is 7.2 to 7.6.
[7] The production method according to any one of [1] to [6], wherein the mixture has fluidity.

### [Advantageous Effects of the Invention]

According to the present invention, a technique for producing a three-dimensional cell structure having a sufficient thickness and a vascular network can be provided.

### [Brief Description of the Drawings]

Fig. 1 shows fluorescence micrographs taken in Experimental Example 1.
Fig. 2 is a graph showing the total length of vessels inside the three-dimensional cell structure measured in Experimental Example 1.
Fig. 3 shows fluorescence micrographs taken in Experimental Example 2.
Fig. 4 is a fluorescence micrograph taken in Experimental Example 3.

### [Description of the Embodiments]

In one embodiment, the present invention provides a method of producing a three-dimensional cell structure, including: mixing a cell cluster including at least an endothelial cell, an extracellular matrix component, and a polymer electrolyte to obtain a mixture; removing a liquid part from the mixture to obtain a cell aggregate; and culturing the cell aggregate in a medium to obtain a three-dimensional cell structure with a thickness of greater than 150 µm and having a vascular network, characterized in that the extracellular matrix component is collagen or a collagen analog, and the polymer electrolyte is heparin or a heparin analog having a final concentration of 0.001 mg/mL or higher in the mixture.

According to the production method of the present embodiment, a technique for producing a three-dimensional cell structure having a sufficient thickness and a vascular network can be provided.

Sufficient thickness may be, for example, more than 150 µm, for example, 160 µm or more, for example, 170 µm or more, for example 180 µm or more, for example, 190 µm or more, for example, more than 200 µm, for example, 210 µm or more, for example, 220 µm or more, for example, 230 µm or more, for example, 240 µm or more, for example, 250 µm or more, for example, 260 µm or more, for example, 270 µm or more, for example, 280 µm or more, for example, 290 µm or more, for example, 300 µm or more. Although the upper limit of the thickness is not particularly limited, it may be, for example, 3000 µm, 1000 µm, or 500 µm. The upper and lower limits of the thickness of the three-dimensional cell structure can be combined as appropriate. The thickness is preferably more than 150 µm and 3000 µm or less, and more preferably 150 µm or more and 500 µm or less.

The total length of the vessels forming the vascular network of the three-dimensional cell structure produced by the production method of the present embodiment may be 5,000 µm/mm² or larger, for example, 5,200 µm/mm² or larger, for example, 5,400 µm/mm² or larger. Although the total length of the vessels is not particularly limited, it may be 5,500 µm/mm², 8,000 µm/mm², or 14,000 µm/mm². The upper and lower limits of the total length of the vessels can be combined as appropriate. The total length is preferably 5,000 µm/mm² or more and 20,000 µm/mm² or less, more preferably 5,000 µm/mm² or more and 10,000 µm/mm² or less.

The total length of vessels is measured as the total length of vessels per unit area of the three-dimensional cell structure when the three-dimensional cell structure is observed from above. The total length of the vessels forming the vascular network can be measured by analyzing a fluorescence micrograph of the three-dimensional cell structure as viewed from above to count the number of fluorescent pixels which represent the vascular network.

The form of the three-dimensional cell structure is not particularly limited. For example, it may be a three-dimensional cell structure formed by culturing cells in a scaffold of a natural biopolymer such as collagen or a synthetic polymer, a cell aggregate (also referred to as a spheroid), or a sheet-like cell structure.

The term "three-dimensional cell structure" as used in the present specification means a three-dimensional cell aggregate including at least one type of cell. Examples of the form of the three-dimensional cell structure are, but not limited to, biological tissue models such as skin, hair, bone, cartilage, teeth, corneas, blood vessels, lymph vessels, heart, liver, pancreas, nerves, and the esophagus, as well as solid cancer models such as gastric cancer, esophageal cancer, colorectal cancer, colon cancer, rectal cancer, pancreatic cancer, breast cancer, ovarian cancer, prostate cancer, renal cell cancer, and liver cancer.

The production method of the present embodiment includes a step (A) of mixing a cell cluster including at least endothelial cells, an extracellular matrix component, and a polymer electrolyte to obtain a mixture, a step (B) of removing the liquid part from the mixture to obtain a cell aggregate, and a step (C) of culturing the cell aggregate in a medium to obtain a three-dimensional cell structure.

In step (A), the cell cluster includes at least endothelial cells. The endothelial cells are preferably vascular endothelial cells. The origin of the cells is not particularly limited, and, for example, they may originate from a human, monkey, dog, cat, rabbit, pig, cow, mouse, or rat.

The cells other than the endothelial cells are not particularly limited, and may be, for example, somatic cells derived from bone, muscle, viscus, nerve, brain, bone, skin, blood, or the like, or germ cells. The cells may also be induced pluripotent stem cells (iPS cells) or embryonic stem cells (ES cells). Alternatively, the cells may be cultured cells such as primary cultured cells, subcultured cells, and cell line cells. The cell cluster may include one type of cell or a plurality of types of cells.

Examples of the cells include, but not limited to, neuronal cells, dendritic cells, immune cells, vascular endothelial cells, lymphatic endothelial cells, fibroblast cells, cancer cells such as liver cancer cells, epithelial cells, myocardial cells, liver cells, pancreatic islet cells, tissue stem cells and smooth muscle cells.

In step (A), a cell cluster including at least endothelial cells, an extracellular matrix component, and a polymer electrolyte are mixed to obtain a mixture. The presence of the extracellular matrix component in the mixture tends to increase the thickness of the cell structure. The mixture may further include a cationic substance.

The extracellular matrix component may be collagen or a collagen analog. Examples of collagen include, but are not limited to, type I collagen, type II collagen, type III collagen, type IV collagen, type V collagen, type VI collagen, type VII collagen, and type VIII collagen. Type I collagen is particularly preferable.

The collagen analog may be a protein having a "collagen-like domain" which partially has the characteristics of a collagen protein, a partial peptide thereof, or the like. More specifically, the collagen analog may be C1q, a collectin, ficolin, adiponectin, macrophage scavenger receptor, or partial peptide thereof. The collagen analog may be an artificial synthetic peptide as long as it partially has the characteristics of a collagen protein.

Examples of heparin or heparin analogs include heparin, heparan sulfate, and Hirudoid (registered trademark) which is a heparinoid. The inclusion of heparin or a heparin analog in the mixture tends to improve the inhibition of vascular network formation by the extracellular matrix.

In step (A), a cell cluster including at least endothelial cells, collagen or a collagen analog, and heparin or a heparin analog are mixed to obtain a mixture.
The final concentration of collagen or a collagen analog in the mixture is, for example, 0.01 mg/mL or higher, for example, 0.025 mg/mL or higher, for example, 0.05 mg/mL or higher, for example, 0.1 mg/mL or higher, for example, 0.15 mg/mL or higher, for example, 0.3 mg/mL or higher. Although the upper limit of the final concentration of collagen or a collagen analog is not particularly limited, it may be, for example, 1.5 mg/mL, 2.0 mg/mL, 3.0 mg/mL, 5.0 mg/mL, 10.0 mg/mL, or 20.0 mg/mL. The upper and lower limits of the final concentration of collagen or a collagen analog in the mixture can be combined as appropriate. For example, the final concentration of collagen or a collagen analog in the mixture may be 0.01 mg/mL or higher and 20.0 mg/mL or lower, 0.01 mg/mL or higher and 10.0 mg/mL or lower, 0.01 mg/mL or higher and 5.0 mg/mL or lower, 0.01 mg/mL or higher and 3.0 mg/mL or lower, 0.01 mg/mL or higher and 2.0 mg/mL or lower, 0.025 mg/mL or higher and 1.5 mg/mL or lower, 0.05 mg/mL or higher and 1.5 mg/mL or lower, 0.05 mg/mL or higher and 3.0 mg/mL or lower.

The final concentration of heparin or a heparin analog in the mixture is 0.001 mg/mL or higher, for example, higher than 0.001 mg/mL, for example, 0.01 mg/mL or higher, for example, 0.025 mg/mL or higher, for example, 0.05 mg/mL or higher, for example, 0.1 mg/mL or higher, for example, 0.15 mg/mL or higher, for example, 0.3 mg/mL or higher, for example, 1.0 mg/mL or higher, for example, 5.0 mg/mL or higher. When the final concentration of heparin or a heparin analog is 0.001 mg/mL or higher, collagen gelation can be prevented. Although the upper limit of the final concentration of heparin or a heparin analog in the mixture is not particularly limited, it may be, for example, 10.0 mg/mL or 50.0 mg/mL. The upper and lower limits of the final concentration of heparin or a heparin analog in the mixture can be combined as appropriate. For example, the final concentration of heparin or a heparin analog in the mixture may be 0.001 mg/mL or higher and 50 mg/mL or lower, 0.001 mg/mL or higher and 10 mg/mL or lower, 0.01 mg/mL or higher and 10 mg/mL or lower, 0.025 mg/mL or higher and 10.0 mg/mL or lower, 0.05 mg/mL or higher and 10.0 mg/mL or lower, 0.001 mg/mL or higher and 0.1 mg/mL or lower, 0.001 mg/mL or higher and lower than 0.025 mg/mL. When the final concentration of heparin or a heparin analog in the mixture is lower than 0.025 mg/mL, collagen gelation can be prevented, and also it is possible to prevent the collagen from becoming too soft.

The final concentration of collagen or a collagen analog and the final concentration of heparin or a heparin analog in the mixture can be combined as appropriate. For example, the final concentration of collagen or a collagen analog in the mixture may be 0.01 mg/mL or higher and 10.0 mg/mL or lower and the final concentration of heparin or a heparin analog may be 0.001 mg/mL or higher and 50.0 mg/mL or lower, the final concentration of collagen or a collagen analog may be 0.05 mg/mL or higher and 1.5 mg/mL or lower and the final concentration of heparin or a heparin analog may be 0.001 mg/mL or higher and 10.0 mg/mL or lower, the final concentration of collagen or a collagen analog may be 0.01 mg/mL or higher and 3.0 mg/mL or lower and the final concentration of heparin or a heparin analog may be 0.001 mg/mL or higher and 10.0 mg/mL or lower.

As the cationic substance, any substance having a positive charge can be used as long as it does not adversely affect the growth of cells and the formation of cell aggregate. Examples of cationic substances include, but are not limited to, cationic buffer solutions such as a tris-hydrochloric acid buffer solution, tris-maleate buffer solution, bis-tris-buffer solution, and HEPES; ethanolamine, diethanolamine, triethanolamine, polyvinylamine, polyallylamine, polylysine, polyhistidine, and polyarginine. Among them, a cationic buffer solution is particularly preferable, and a tris-hydrochloric acid buffer solution is even more preferable.

The final concentration of the cationic substance in the mixture in step (A) is not particularly limited as long as it does not adversely affect the growth of cells and the formation of cell aggregate. The final concentration of the cationic substance used in this embodiment is preferably 10 to 100 mM, and, for example, it may be 20 to 90 mM, for example, 30 to 80 mM, for example, 40 to 70 mM, for example, 45 to 60 mM.

In the case a cationic buffer solution is used as the cationic substance, the pH of the cationic buffer solution is not particularly limited as long as it does not adversely affect the growth of cells and the formation of cell aggregate. The pH of the cationic buffer solution used in this embodiment is preferably 6.0 to 8.0. For example, the pH of the cationic buffer solution used in this embodiment may be 7.0, 7.1, 7.2, 7.3, 7.4, 7.5, 7.6, 7.7, 7.8, 7.9, 8.0. To reduce the damage to the cells, the pH of the cationic buffer solution used in this embodiment is more preferably 7.2 to 7.6, more preferably 7.35 to 7.45, and even more preferably 7.4. The pH of the mixture in step (A) can be adjusted to be in the above range by using the cationic buffer solution. Note that the pH specified in the present specification is the value measured at room temperature (20 to 25°C).

In general, collagen is soluble in water under acidic conditions, but is insoluble and gels in water under neutral to alkaline conditions. Therefore, when the pH of the mixture in step (A) is 7.0 to 8.0, the collagen will gel, but when heparin is included in the mixture in a proportion of at least 0.001 mg/mL, collagen gelation can be prevented. That is, the mixture in step (A) has fluidity. This facilitates the movement of endothelial cells in cell culture described later, and thus facilitates the aggregation of the endothelial cells. As a result, a vascular network is likely to be formed in the three-dimensional cell structure.

Whether the mixture has fluidity or not is determined in the present specification using the following method. First, a cell cluster including at least endothelial cells, an extracellular matrix component, and a polymer electrolyte are mixed in a 1.5 mL tube to prepare a mixture. After leaving it overnight at 4°C, the tube is incubated at 37°C for 30 minutes. The mixture in the tube is observed with the naked eye. The tube is inverted, and if the mixture in the tube flows after 1 second, it is determined to be "fluid", and "not fluid" if it does not flow. Further, the presence or absence of fluidity may also be determined by dynamic viscoelasticity measurement. Specifically, the storage elastic modulus X and the loss elastic modulus Y of the mixture at a temperature that is equal to or higher than room temperature may be measured, and if X is larger than Y, it may be determined that the mixture has gelled and is "not fluid", and if X is smaller than Y, it may be determined that the mixture has not gelled and is "fluid". Note that, since the proportion of a cell cluster in the mixture is extremely small, it is assumed that the determination on fluidity is not affected even when the mixture does not contain a cell cluster.

When the mixture of step (A) includes heparin in a proportion of at least 0.001 mg/mL, collagen is uniformly distributed in the mixture. As a result, the uniformity of cell distribution in the mixture can also be improved.

In step (A), the collagen or collagen analog, and the heparin or heparin analog can be used by dissolving them in a suitable solvent. Examples of solvents include, but are not limited to, water, an acidic aqueous solution, and a buffer solution. The collagen or collagen analog may be dissolved in an acidic aqueous solution, for example, an acetic acid aqueous solution. in the case a cationic buffer solution is used as the above-mentioned cationic substance, collagen or a collagen analog, and heparin or a heparin analog may be used by dissolving them in the cationic buffer solution.

In step (A), the mixing of the cell cluster with collagen or a collagen analog, heparin or a heparin analog, and optionally a cationic substance may be carried out in a suitable container such as a dish, tube, flask, bottle, or plate.

Subsequently, in step (B), the liquid part is removed from the mixture to obtain a cell aggregate. The term "cell aggregate" as used in the present specification means a cluster of cells. The cell aggregate also includes a cell precipitate obtained by centrifugation, filtration, or the like. In an embodiment, the cell aggregate is a slurry-like viscous body. The term "slurry-like viscous body" refers to a gel-like cell aggregate like the one described in Akihiro Nishiguchi et al., Cellcell crosslinking by bio-molecular recognition of heparin-based layer-by-layer nanofilms, Macromol Biosci., 15 (3), 312-317, 2015.

The liquid part can be removed using a method known to those skilled in the art. For example, the liquid part may be removed by centrifugation or filtration. The conditions of centrifugation are not particularly limited as long as they do not adversely affect the growth of cells and the formation of cell aggregate. For example, the liquid part may be removed by centrifuging a microtube containing the mixture at 400 to 1,000 × g at room temperature for one minute to separate the liquid part from the cell aggregate. Alternatively, the liquid part may be removed after collecting the cells using spontaneous sedimentation. As a result, a cell aggregate can be obtained.

Subsequently, in step (C), the cell aggregate is cultured in a medium to obtain a three-dimensional cell structure. The cell aggregate may be suspended in a solution prior to the culturing. The solution is not particularly limited as long as it does not adversely affect the growth of cells and the formation of a three-dimensional cell structure. For example, a cell culture medium, a buffer solution, or the like suitable for the cells constituting the cell aggregate can be used. The suspension of the cell aggregate can be carried out in a suitable container such as a dish, tube, flask, bottle, or plate.

When the cell aggregate is suspended in a solution, it is also possible to precipitate the cells and form a cell deposit on a substrate before the culturing. The substrate may be a culture vessel used to culture cells. The culture vessel may be a vessel having a material and a shape normally used in cell or microorganism culture. Examples of the material of the culture vessel include, but are not limited to, glass, stainless steel, and plastic. Examples of culture vessels include, but are not limited to, a dish, tube, flask, bottle, and plate. The substrate is, for example, a material that does not allow cells in the liquid to pass through but allows the liquid to pass through. The substrate is preferably a permeable membrane. Examples of vessels having such permeable membrane include, but are not limited to, cell culture inserts such as Transwell (registered trademark) inserts, Netwell (registered trademark) inserts, Falcon (registered trademark) cell culture inserts, and Millicell (registered trademark) cell culture inserts.

A method known to those skilled in the art can be used to precipitate the cells. For example, the cells may be collected by centrifugation, magnetic separation, filtration, or the like. The conditions of centrifugation are not particularly limited as long as they do not adversely affect the growth of cells. For example, the cells may be collected by seeding the mixture or suspension in a cell culture insert and centrifuging it at 400-1,000 x g at 10°C or room temperature for one minute. Alternatively, the cells may be collected using spontaneous sedimentation. Further, the collected cells may form a layer structure.

The cell aggregate, or, in the case the cell aggregate is suspended, the suspended cells is/are preferably seeded at a cell density of 1,000 cells/mm² or higher, for example, 10,000 cells/mm² or higher, for example, 20,000 cells/mm² or higher, for example, 25,000 cells/mm² or higher, with respect to the area of the culture vessel. When the number of cells with respect to the area of the culture vessel is 1,000 cells/mm² or more, the cells are appropriately dispersed, and the thickness of the three-dimensional cell structure tends to exceed 150 µm. In addition, this facilitates the aggregation of the endothelial cells since the endothelial cells can move easily. Further, the cells may be seeded at a cell density of, for example, 1,000,000 cells/mm² or lower, for example, 500,000 cells/mm² or lower, for example, 200,000 cells/mm² or lower, for example, 100,000 cells/mm² or lower, with respect to the area of the culture vessel. When the number of cells with respect to the area of the culture vessel is 1,000,000 cells/mm² or less, it is possible to prevent the three-dimensional cell structure from becoming thin. The upper and lower limits of the cell density can be combined as appropriate. For example, the cell density with respect to the area of the culture vessel may be 1,000 cells/mm² or higher and 100,000 cells/mm² or lower, or for example 10,000 cells/mm² or higher and 200,000 cells/mm² or lower. By performing the step of suspending the cell aggregate in a solution and the step of cell deposition, a more homogeneous three-dimensional cell structure can be obtained.

In step (C), cell culture can be carried out under culture conditions that are suitable for the cells to be cultured. Those skilled in the art can select an appropriate medium according to the cell type and desired function. The cell culture medium is not particularly limited, but it may be a basic medium such as DMEM, E-MEM, MEMα, RPMI-1640, Mccoy'5a, or Ham's F-12, or a medium obtained by adding CS (also called bovine serum), FBS (also called fetal bovine serum), or HBS (also called fetal horse serum) to a basic medium so that the serum content is about 1 to 20% by volume. Conditions such as the temperature of the culture environment and the atmospheric composition can also be easily determined by those skilled in the art.

Upon cell culture, a substance for suppressing deformation of the constructed three-dimensional cell structure (for example, tissue contraction, peeling at the tissue edge, or the like) may be added to the medium. An example of such a substance is Y-27632, which is a selective ROCK (also called Rho-associated coiled-coil forming kinase/Rho-binding kinase) inhibitor, but the substance is not limited to this.

It is also possible to form a laminate of cell aggregates or cell deposits by repeating the above steps. In other words, steps (A) and (B) may be repeated a plurality of times, and step (C) may be performed after that. This makes it possible to construct a three-dimensional cell structure having a plurality of layers. However, according to the production method of the present embodiment, a three-dimensional cell structure having a thickness that is greater than 150 µm, preferably greater than 200 µm can be obtained without repeating the above steps.

In the case the above steps are repeated to form a laminate of cell aggregates or cell deposits, a plurality of types of cells may be used to construct a three-dimensional cell structure composed of different types of cells. Specifically, step (A) is performed using a cell cluster including a first cell and a second cell different from the first cell as the cell cluster (referred to as step (A1)), and then step (B) is performed. A three-dimensional cell structure may be constructed by repeating steps (A1) and (B), and performing step (C) after that. As another aspect, step (A) is performed using a cell cluster including the first cell as the cell cluster (referred to as step (A2)), and step (B) is performed. Then, step (A) is performed using a cell cluster including a second cell different from the first cell as the cell cluster (referred to as step (A3)), and then step (B) is performed. A three-dimensional cell structure may be constructed by repeating the combination of steps (A2) and (B) and the combination of steps (A3) and (B) a plurality of times, and then performing step (C). Note that, in the above production method, it is possible to continuously perform the combination of steps (A2) and (B) a plurality of times, and then perform the combination of steps (A3) and (B), or continuously perform the combination of steps (A3) and (B) a plurality of times, and then perform the combination of steps (A2) and (B).

Since the three-dimensional cell structure produced by the production method of the present embodiment has a vascular network, it is useful as a model imitating a structure that is close to a living body. The term "vascular network" as used in the present specification refers to a network structure having a number of branching points, such as a blood vessel network or a lymphatic vessel network, in living tissues.

It can be said that the production method of the present embodiment is a method of providing a vascular network to a thick three-dimensional cell structure. Further, in the production method of the present embodiment, it can be said that the heparin or heparin analog is a vascular network forming agent for forming a vascular network in a cell structure. It can also be said that heparin or a heparin analog is an improving agent for improving the density of the vascular network formed in the three-dimensional cell structure.

The present invention encompasses the following modes as another aspect.
[8] A method of producing a three-dimensional cell structure, including: mixing a cell cluster including at least an endothelial cell, an extracellular matrix component, and a polymer electrolyte to obtain a mixture; removing a liquid part from the mixture to obtain a cell aggregate; and culturing the cell aggregate in a medium to obtain a three-dimensional cell structure with a thickness of greater than 150 µm and having a vascular network, characterized in that the extracellular matrix component is collagen or a collagen analog, and the polymer electrolyte is heparin or a heparin analog having a final concentration of 0.001 mg/mL or higher and 0.05 mg/mL or lower, or 0.001 mg/mL or higher and lower than 0.025 mg/mL in the mixture.
[9] The production method according to [8], characterized in that, in the culturing of the cell aggregate in a medium, the cell aggregate is seeded at a cell density of 20,000 cells/mm² to 100,000 cells/mm² with respect to the area of a culture vessel.
[10] The production method according to [8] or [9], wherein a total length of vessels constituting the vascular network is 5,000 µm/mm² or more and 10,000 µm/mm² or less.
[11] The production method according to any one of [8] to [10], wherein the endothelial cell is a vascular endothelial cell.
[12] The production method according to any one of [8] to [11], wherein the cell aggregate further includes a fibroblast.
[13] The production method according to any one of [8] to [12], wherein a pH of the mixture is 7.35 to 7.45.
[14] The production method according to any one of [8] to [13], wherein the mixture has fluidity.
[15] The production method according to any one of [8] to [14], characterized in that a final concentration of the extracellular matrix component in the mixture is 0.01 mg/mL or higher and 3.0 mg/mL or lower (preferably 0.01 mg/mL or higher and 1.5 mg/mL or lower).

### [Examples]

In the following, the present invention will be described in more detail and more specifically by way of examples. However, the examples should not limit the scope of the claims of the present invention.

In the following examples, collagen I was used as the collagen unless otherwise specified.

### [Experimental Example 1]

### (Three-dimensional cell structure formation 1)

4.25 × 10⁵ normal human skin fibroblasts (NHDF) and 7.0 × 10³ GFP-introduced human umbilical vein endothelial cells (GFP-HUVEC) were suspended in a mixture containing equal amounts of a 0 mg/mL, 0.2 mg/mL, or 2.0 mg/mL heparin/50 mM tris-hydrochloric acid buffer solution (pH 7.4) and a 0 mg/mL, 0.2 mg/mL, or 0.6 mg/mL collagen/5 mM acetic acid solution (pH 3.7). As a result, the final concentration of collagen in the mixture became 0 mg/mL, 0.1 mg/mL, or 0.3 mg/mL, respectively, and the final concentration of heparin became 0 mg/mL, 0.1 mg/mL, or 1.0 mg/mL, respectively.

Subsequently, the obtained mixture was centrifuged at 1,000 × g (gravitational acceleration) at room temperature for one minute to obtain a viscous body. The obtained viscous body was suspended in DMEM containing 10% fetal bovine serum (FBS). The entire amount of the obtained suspension was seeded in a 96-well cell culture insert (bottom area per well was 0.143 cm²) and centrifuged at 400 × g at room temperature for one minute. As a result, a cell layer was formed on the cell culture insert. The density of cells seeded in the cell culture insert was 29,720 cells/mm².

Subsequently, DMEM containing 10% FBS was added so that the total liquid volume inside and outside the cell culture insert became greater than 2 mL, and the cells were cultured in a CO₂ incubator (37°C, 5% O₂) (the time at which this culture was started is referred to as the culture start time). After culturing for 24 hours from the culture start time, the medium was replaced with DMEM containing 10% FBS. Subsequently, the medium was also changed 48 and 96 hours after the culture start time.

### <<Observation of three-dimensional cell structure>>

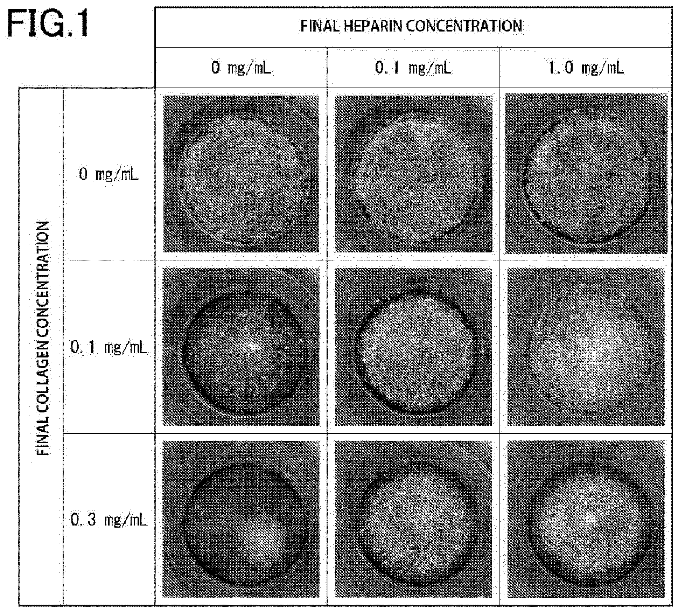

120 hours after the culture start time, the three-dimensional cell structure was observed from above in a living state with a fluorescence microscope, and the fluorescence of GFP was detected. Fig. 1 shows photographs showing the results of observation under a fluorescence microscope. As a result, in the samples with a final collagen concentration of 0.1 mg/mL or higher and a final heparin concentration of 0 mg/mL, the formation of the blood vessel network was inhibited as compared with the other samples. On the other hand, in the samples with a final heparin concentration of 0.1 mg/mL or higher, even when the final collagen concentration was 0.1 mg/mL or higher, the inhibition of blood vessel network formation was significantly alleviated.

### <<Measurement of thickness of three-dimensional cell structure>>

Subsequently, the thickness of each three-dimensional cell structure was measured. The height measurement was calculated by subtracting the height of the bottom surface of the 96-well cell culture insert from the height at which the confocal microscope was focused on the target.

The following Table 1 shows the measurement results of the thickness of the thirddimensional cell structure. In Table 1, "N.D." indicates that the three-dimensional cell structure had contracted and the thickness of the three-dimensional cell structure could not be measured. The thickness resolution was 50 µm because the focal length of the lens of the confocal microscope used was about 25 µm, and, if two points in the height direction are separated by 50 µm, the beam cannot be focused on both of the points.

**[Table 1]**

| Final collagen concentration (mg/mL) | Final heparin concentration (mg/mL) | Thickness of three-dimensional structure (µm) |
|---|---|---|
| 0 | 0 | 50-100 |
| 0 | 0.1 | 50-100 |
| 0 | 1.0 | 100-150 |
| 0.1 | 0 | N.D. |
| 0.1 | 0.1 | 150-200 |
| 0.1 | 1.0 | 200-250 |
| 0.3 | 0 | N.D. |
| 0.3 | 0.1 | 200-250 |
| 0.3 | 1.0 | 250-300 |

As a result, it was found that, in the samples with a final collagen concentration of 0.1 mg/mL or higher and a final heparin concentration higher than 0.1 mg/mL, the thickness of the three-dimensional cell structure could be greater than 150 µm.

### <<Measurement of total length of vessels>>

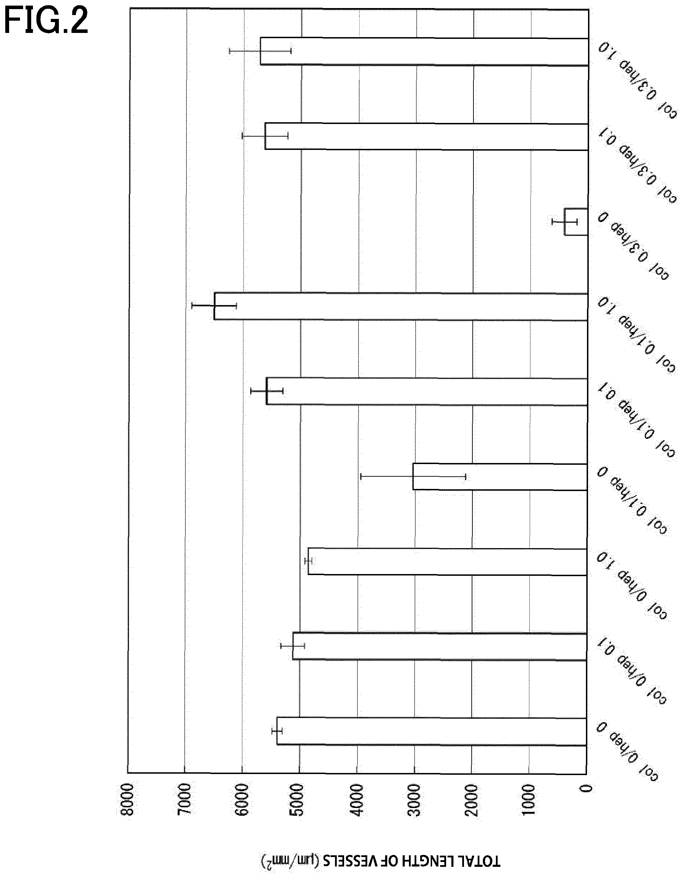

Subsequently, the total length of the vessels constituting the vascular network inside each three-dimensional cell structure was measured by image analysis of the fluorescence micrographs shown in Fig. 1. Fig. 2 is a graph showing the measured total length of vessels. In Fig. 2, "col" indicates collagen and "hep" indicates heparin. Further, "col 0/hep 0" indicates that it is the result of the sample with a final collagen concentration of 0 mg/mL and a final heparin concentration of 0 mg/mL, "col 0/hep 0.1" indicates that it is the result of the sample with a final collagen concentration 0 mg/mL and a final heparin concentration of 0.1 mg/mL, and so on.

As a result, it was found that, in the samples with a final collagen concentration of 0.1 mg/mL or higher and a final heparin concentration higher than 0.1 mg/mL, the total length of vessels could be 5,000 µm/mm² or more.

### [Experimental Example 2]

### (Three-dimensional cell structure formation 2)

This experimental example mainly differed from Experimental Example 1 in the final collagen concentration, the final heparin concentration, and the cell culture time. First, 4.25 × 10⁵ normal human skin fibroblasts (NHDF) and 4.5 × 10³ GFP-introduced human umbilical vein endothelial cells (GFP-HUVEC) were suspended in a mixture containing equal amounts of a 0 mg/mL, 0.002 mg/mL, 0.2 mg/mL, or 10.0 mg/mL heparin/50 mM tris-hydrochloric acid buffer solution (pH 7.4) and a 0 mg/mL or 0.3 mg/mL collagen/5 mM acetic acid solution (pH 3.7). As a result, the final concentration of collagen in the mixture became 0 mg/mL or 0.15 mg/mL, respectively, and the final concentration of heparin became 0 mg/mL, 0.001 mg/mL, 0.1 mg/mL, or 5.0 mg/mL, respectively. The experimental example with a final collagen concentration of 0 mg/mL was carried out only for a final heparin concentration of 0 mg/mL.

Then, each of the obtained mixtures was centrifuged at 1,000 × g at room temperature for one minute to obtain a viscous body. The obtained viscous body was suspended in DMEM containing 10% FBS. The entire amount of the obtained suspension was seeded in a 96-well cell culture insert and subjected to 400 × g centrifugation at room temperature for one minute. As a result, a cell layer was formed on the cell culture insert. The density of cells seeded in the cell culture insert was 29,550 cells/mm².

Subsequently, DMEM containing 10% FBS was added so that the total liquid volume inside and outside the cell culture insert was greater than 2 mL, and the cells were cultured in a CO₂ incubator (37°C, 5% O₂) (the time at which this culture was started is referred to as the culture start time). After culturing for 24 hours from the culture start time, the medium was replaced with DMEM containing 10% FBS. Subsequently, the medium was also changed 48, 96, and 144 hours after the culture start time.

### <<Observation of three-dimensional cell structure>>

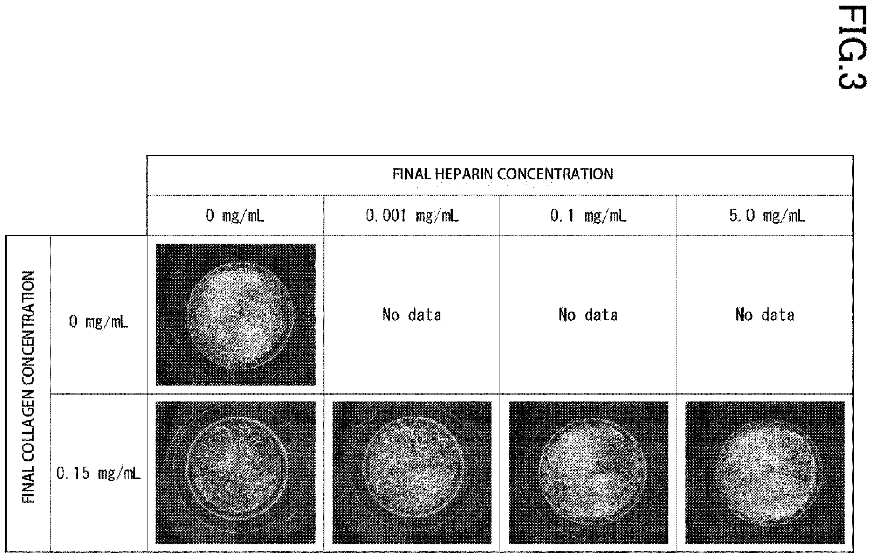

168 hours after the culture start time, the three-dimensional cell structure was observed from above in a living state with a fluorescence microscope, and the fluorescence of GFP was detected. Fig. 3 shows photographs showing the results of observation under a fluorescence microscope. In Fig. 3, "No data" indicates that no micrograph was taken.

As a result, in the sample with a final collagen concentration of 0.15 mg/mL and a final heparin concentration of 0 mg/mL, the formation of the blood vessel network was inhibited as compared with the other samples. On the other hand, in the samples with a final heparin concentration of 0.001 mg/mL or higher, even when the final collagen concentration was 0.15 mg/mL or higher, the inhibition of blood vessel network formation was significantly alleviated.

### [Experimental Example 3]

### (Three-dimensional cell structure formation 3)

A three-dimensional cell structure was prepared similarly to Experimental Example 1 except that collagen with a final concentration of 0.1 mg/mL was used as the extracellular matrix component and hyaluronic acid with a final concentration of 0.1 mg/mL was used as the polymer electrolyte.

### <<Observation of three-dimensional cell structure>>

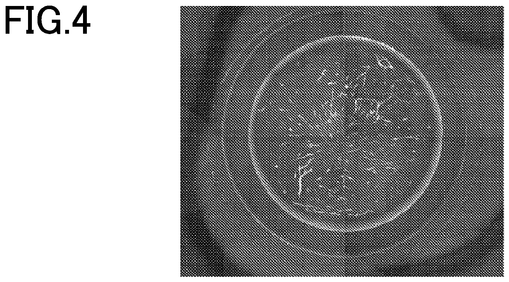

120 hours after the culture start time, the three-dimensional cell structure was observed from above in a living state with a fluorescence microscope, and the fluorescence of GFP was detected. Fig. 4 shows photographs showing the results of observation under a fluorescence microscope. As a result, it was found that, when hyaluronic acid is used as the polymer electrolyte, the inhibition of the blood vessel network formation by collagen will not be alleviated.

### [Experimental Example 4]

### (Mixture fluidity check)

A mixture containing equal amounts of a 0 mg/mL, 0.002 mg/mL, 0.05 mg/mL, 0.2 mg/mL or 2.0 mg/mL heparin/50 mM or 200 mM tris-hydrochloric acid buffer solution and a 0.2 mg/mL or 0.3 mg/mL collagen/5 mM acetic acid solution was prepared in a 1.5 mL tube. As a result, as described in conditions 1 to 8 shown in Table 2, the final concentration of collagen in the mixture was 0.1 mg/mL or 0.15 mg/mL, respectively, and the final concentration of heparin was 0 mg, 0.001 mg/mL, 0.025 mg/mL, 0.1 mg/mL, or 1.0 mg/mL, respectively.

The pH of 1 mL of each of the mixtures of conditions 1 to 8 was measured. The tube containing the mixture was left overnight at 4°C. After incubating the tube at 37°C for 30 minutes, the tube was inverted to visually confirm whether the mixture in the tube flows after 1 second. When the mixture flowed, the sample was determined to be "fluid", and "not fluid" when it did not. Table 2 shows the pH and fluidity of the mixture under each condition.

**[Table 2]**

| Condition | 1 | 2 | 3 | 4 | 5 | 6 | 7 | 8 |
|---|---|---|---|---|---|---|---|---|
| Acetic acid | 2.5 mM | 2.5 mM | 2.5 mM | 2.5 mM | 2.5 mM | 2.5 mM | 2.5 mM | 2.5 mM |
| Collagen | 0.15 mg/mL | 0.15 mg/mL | 0.15 mg/mL | 0.1 mg/mL | 0.1 mg/mL | 0.1 mg/mL | 0.1 mg/mL | 0.1 mg/mL |
| Heparin | - | - | 0.5 mg/mL | - | 0.001 mg/mL | 0.025 mg/mL | 0.1 mg/mL | 1.0 mg/mL |
| Tris | 25 mM | 100 mM | 100 mM | 100 mM | 100 mM | 100 mM | 100 mM | 100 mM |
| pH | 6.96 | 7.38 | 7.41 | 7.38 | 7.38 | 7.41 | 7.40 | 7.41 |
| Fluidity after incubation | Fluid | Not fluid | Fluid | Not fluid | Fluid | Fluid | Fluid | Fluid |

Under condition 1, the pH was 6.96, and the mixture had fluidity even though it did not include heparin. From the results of conditions 3 and 5 to 8, it was found that, when the mixture includes heparin, the mixture has fluidity even when its pH is 7.38 or higher, and thus is suitable for the preparation of a three-dimensional cell structure having a vascular network. From the result of condition 5, it was confirmed that the fluidity of the mixture is improved even when the heparin concentration is 0.001 mg/mL. Further, from the results of conditions 3 and 5 to 8, it was found that the fluidity of the mixture is improved even if the heparin concentration in the mixture changes with respect to the collagen concentration.

On the other hand, under conditions 2 and 4 which specify that no heparin is present and the pH is 7.38, the mixture gelled and had no fluidity.

### [Industrial Applicability]

According to the present invention, a technique for producing a three-dimensional cell structure having a sufficient thickness and a vascular network can be provided.

## Claims

1. A method of producing a three-dimensional cell structure, comprising:
mixing a cell cluster including at least an endothelial cell, an extracellular matrix component, and a polymer electrolyte to obtain a mixture;
removing a liquid part from the mixture to obtain a cell aggregate; and
culturing the cell aggregate in a medium to obtain a three-dimensional cell structure with a thickness greater than 150 µm and having a vascular network, **characterized in that**
the extracellular matrix component is collagen or a collagen analog, and
the polymer electrolyte is heparin or a heparin analog having a final concentration of 0.001 mg/mL or higher in the mixture.

2. The production method according to claim 1, **characterized in that**, in the culturing of the cell aggregate in a medium, the cell aggregate is seeded at a cell density of 1,000 cells/mm² to 1,000,000 cells/mm² with respect to the area of a culture vessel.

3. The production method according to claim 1 or 2, **characterized in that** a total length of vessels constituting the vascular network is 5,000 µm/mm² or more.

4. The production method according to any one of claims 1 to 3, **characterized in that** the endothelial cell is a vascular endothelial cell.

5. The production method according to any one of claims 1 to 4, **characterized in that** the cell aggregate further includes a fibroblast.

6. The production method according to any one of claims 1 to 5, wherein a pH of the mixture is 7.2 to 7.6.

7. The production method according to any one of claims 1 to 6, wherein the mixture has fluidity.
